(19) Europäisches Patentamt
European Patent Office
Office européen des brevets

(11) Veröffentlichungsnummer: **0 433 809 A1**

(12) **EUROPÄISCHE PATENTANMELDUNG**

(21) Anmeldenummer: **90123611.7**

(51) Int. Cl.[5]: **C07C 275/30**, A01N 47/30

(22) Anmeldetag: **08.12.90**

(30) Priorität: **22.12.89 DE 3942462**

(43) Veröffentlichungstag der Anmeldung:
**26.06.91 Patentblatt 91/26**

(84) Benannte Vertragsstaaten:
**BE CH DE FR GB IT LI NL**

(71) Anmelder: **BAYER AG**
**W-5090 Leverkusen 1 Bayerwerk(DE)**

(72) Erfinder: **Haug, Michael, Dr.**
**Fahner Weg 5**
**W-5060 Bergisch Gladbach 2(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch-Gladbach 2(DE)**

(54) **Difluorphenylharnstoffe.**

(57) Die Erfindung betrifft neue Difluorphenylharnstoffe der Formel (I)

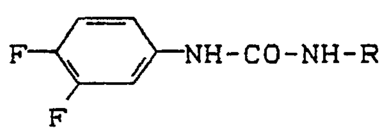

(I)

in welcher

R für jeweils geradkettiges oder verzweigtes Alkyl, Alkenyl oder Alkinyl mit jeweils bis zu 6 Kohlenstoffatomen, für jeweils gegebenenfalls durch Alkylreste substituiertes Cycloalkyl oder Cycloalkylalkyl mit bis zu 6 Kohlenstoffatomen im Cycloalkylteil und gegebenenfalls bis zu 4 Kohlenstoffatomen im Alkylteil, oder für jeweils geradkettiges oder verzweigtes Alkoxy oder Alkenyloxy mit jeweils bis zu 6 Kohlenstoffatomen steht,

ein Verfahren zu ihrer Herstellung und ihre Verwendung als Herbizide.

EP 0 433 809 A1

## DIFLUORPHENYLHARNSTOFFE

Die Erfindung betrifft neue Difluorphenylharnstoffe, ein Verfahren zu ihrer Herstellung und ihre Verwendung als Herbizide.

Es ist bekannt, daß bestimmte Fluorphenylharnstoffe, wie z.B. 1-Methyl-3-(4-fluor-phenyl)-harnstoff, 1,1-Dimethyl-3-(4-fluor-phenyl)-harnstoff und 1,1-Dimethyl-3-(3,4-difluor-phenyl)-harnstoff, herbizide Eigenschaften aufweisen (vgl. DE-OS 19 05 599 und DE-OS 38 00 269).

Die Herbizidwirkung dieser bekannten Verbindungen bzw. ihre Kulturpflanzen-Verträglichkeit ist jedoch nicht immer ganz zufriedenstellend.

Es wurden nun die neuen Difluorphenylharnstoffe der Formel (I)

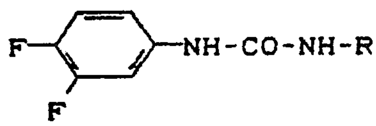

(I)

in welcher

R für jeweils geradkettiges oder verzweigtes Alkyl, Alkenyl oder Alkinyl mit jeweils bis zu 6 Kohlenstoffatomen, für jeweils gegebenenfalls durch Alkylreste substituiertes Cycloalkyl oder Cycloalkylalkyl mit bis zu 6 Kohlenstoffatomen im Cycloalkylteil und gegebenenfalls bis zu 4 Kohlenstoffatomen im Alkylteil, oder für jeweils geradkettiges oder verzweigtes Alkoxy oder Alkenyloxy mit jeweils bis zu 6 Kohlenstoffatomen steht,

gefunden.

Man erhält die neuen Difluorphenylharnstoffe der Formel (I), wenn man das Difluorphenylisocyanat der Formel (II)

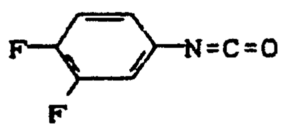

(II)

mit Aminen der Formel (III)

$$H_2N-R \qquad (III)$$

in welcher

R die oben angegebene Bedeutung hat,

gegebenenfalls in Gegenwart eines Verdünnungsmittels umsetzt.

Die neuen Difluorphenylharnstoffe der Formel (I) zeichnen sich durch starke herbizide Wirksamkeit aus.

Überraschenderweise zeigen die neuen Verbindungen der Formel (I) erheblich bessere, insbesondere selektivere Wirkung als vorbekannte Fluorphenylharnstoffe gleicher Wirkungsrichtung.

Weitere mögliche Herstellungsmethoden für die erfindungsgemäßen Verbindungen der Formel (I) sind nachstehend skizziert, wobei R die oben angegebene Bedeutung hat:

(a) Umsetzung von 3,4-Difluoranilin (IV) mit Isocyanaten (V):

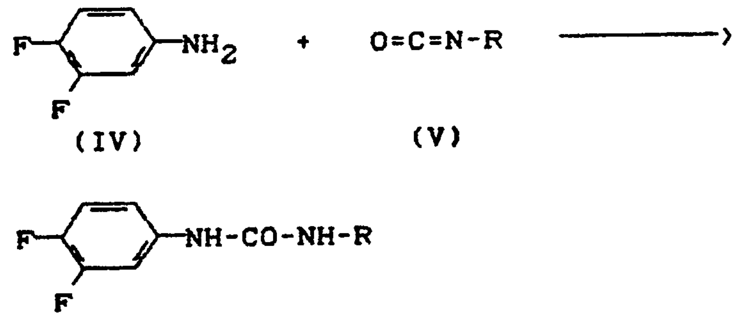

(IV) (V)

(I)

(b) Umsetzung von 3,4-Difluoranilin (IV) mit Chlorameisensäureestern (VI) ($R^1$: $C_1$-$C_4$-Alkyl, Benzyl, Phenyl) und dann mit Aminen (III):

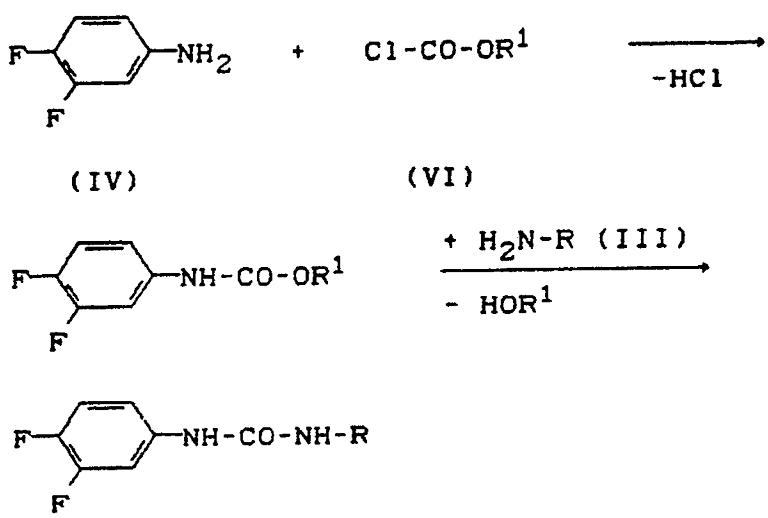


(IV) (VI)

(I)

Die neuen Difluorphenylharnstoffe sind durch die Formel (I) allgemein definiert. Bevorzugt sind Verbindungen der Formel (I), worin

R für jeweils geradkettiges oder verzweigtes Alkyl, Alkenyl oder Alkinyl mit jeweils bis zu 4 Kohlenstoffatomen, für jeweils gegebenenfalls durch $C_1$-$C_4$-Alkylreste substituiertes Cycloalkyl oder Cycloalkylalkyl mit bis zu 6 Kohlenstoffatomen im Cycloalkylteil und gegebenenfalls 1 oder 2 Kohlenstoffatomen im Alkylteil, oder für jeweils geradkettiges oder verzweigtes Alkoxy oder Alkenyloxy mit jeweils bis zu 4 Kohlenstoffatomen steht.

Besonders bevorzugt sind Difluorphenylharnstoffe der Formel (I), worin

R für Methyl, Ethyl, Propyl, Isopropyl, Allyl, Propargyl oder Cyclopropyl steht.

Verwendet man für das erfindungsgemäße Verfahren 3,4-Difluor-phenylisocyanat und Methylamin als Ausgangsstoffe, so kann der Reaktionsablauf durch das folgende Formelschema skizziert werden:

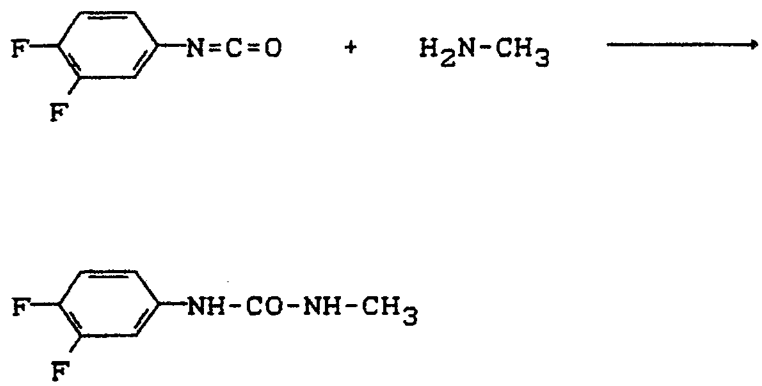

Das beim erfindungsgemäßen Verfahren als Ausgangsstoff zu verwendende 3,4-Difluor-phenylisocyanat der Formel (II) ist bereits bekannt (vgl. DE-OS 27 25 074).

Die weiter als Ausgangsstoffe zu verwendenden Amine sind durch die Formel (III) allgemein definiert. In dieser Formel (III) hat R vorzugsweise bzw. besonders bevorzugt diejenigen Bedeutungen, die bereits oben im Rahmen der Beschreibung der erfindungsgemäßen Verbindungen der Formel (I) als bevorzugt bzw. besonders bevorzugt für R angegeben wurden. Die Ausgangsstoffe der Formel (III) sind bekannte organische Synthesechemikalien.

Das erfindungsgemäße Verfahren zur Herstellung der neuen Difluorphenylharnstoffe der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid

Die Reaktionstemperaturen können bei dem erfindungsgemäßen Verfahren in einem größeren Bereich variiert werden. Im allgemeinen arbeitet man bei Temperaturen zwischen -20°C und +100°C, vorzugsweise bei Temperaturen zwischen -10°C und +50°C.

Das erfindungsgemäße Verfahren wird im allgemeinen unter Normaldruck durchgeführt. Es ist jedoch auch möglich unter erhöhtem oder vermindertem Druck zu arbeiten.

Zur Durchführung des erfindungsgemäßen Verfahrens setzt man je Mol 3,4-Difluor-phenylisocyanat der Formel (II) im allgemeinen zwischen 0,9 und 2,0 Mol, vorzugsweise zwischen 1,0 und 1,5 Mol, Amin der Formel (III) ein.

Die Ausgangsstoffe der Formel (II) und (III) können in beliebiger Reihenfolge zusammengegeben werden.

In einer bevorzugten Ausführungsform des erfindungsgemäßen Verfahrens wird das Difluorphenylisocyanat der Formel (II) in einem geeigneten Verdünnungsmittel vorgelegt und das Amin der Formel (III) wird langsam zudosiert. Das Produkt der Formel (I) fällt im Laufe der Umsetzung im allgemeinen kristallin an und kann durch Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Unkrautbekämpfung, insbesondere zur Bekämpfung von dikotylen Unkräutern, in monokotylen und dikotylen Kulturen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff-(METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner

auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl} -benzolsulfonamid (CHLORSULFURON); N,N-Dimethyl-N'-(3-chlor-4-methylphenyl)-harnstoff (CHLORTOLURON); exo-1-Methyl-4-( 1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl )-5-oxo-1H-imidazol-2-yl]-4 (5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Ethyl-6-methyl-N-(1-methyl-2-methoxyethyl)-chloracetanilid (METOLACHLOR); 2-{[[((4-Methoxy-6-methyl-1 ,3,5-triazin-2-yl)-amino)-carbonyl]-amino]-sulfonyl}-benzoesäure oder deren Methylester (METSULFURON); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitrophenyl)-ether (OXYFLUORFEN); N-(1-Ethylpropyl)-3,4-dimethyl-2,6-dinitroanilin (PENDIMETHALIN); 0-(6-Chlor-3-phenyl-pyridazin-4-yl)-S-octyl-thiolcarbamat(PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN); einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Losungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro ha.

Die Herstellung und die Verwendung der erf indungsgemäßen Wirkstoffe geht aus den nachfolgenden Beispielen hervor.

Herstellungsbeispiele:

Beispiel 1

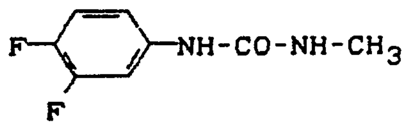

Methylamin wird bis zur Sättigung in eine auf 0°C abgekühlte Mischung aus 15,3 g (0,09 Mol) 3,4-Difluor-phenylisocyanat und 150 ml Toluol eingeleitet. Das kristallin angefallene Produkt wird anschließend durch Absaugen isoliert und aus Acetonitril umkristallisiert.

Man erhält 10,9 g (65% der Theorie) 1-Methyl-3-(3,4-difluor-phenyl)-harnstoff vom Schmelzpunkt 180°C.

Beispiel 2

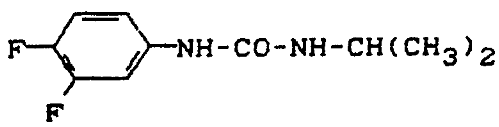

Analog Beispiel 1 erhält man 1-Isopropyl-3-(3,4-difluorphenyl)-harnstoff vom Schmelzpunkt 174°C.

Beispiel 3

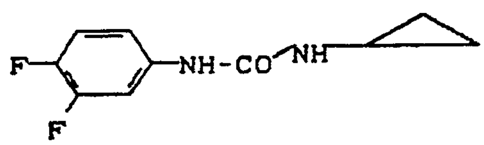

Analog Beispiel 1 erhält man 1-Cyclopropyl-3-(3,4-difluor-phenyl)-harnstoff vom Schmelzpunkt 166°C.

Beispiel 4

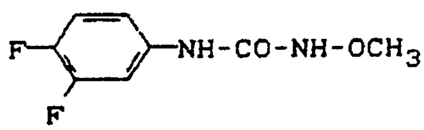

Analog Beispiel 1 erhält man 1-Methoxy-3-(3,4-difluorphenyl)-harnstoff vom Schmelzpunkt 116°C.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der

Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt die gemäß dem Herstellungsbeispiel (1) erhaltene Verbindung bei sehr starker Wirkung gegen Unkräuter eine wesentlich bessere Verträglichkeit gegenüber Kulturpflanzen, wie z.B. Weizen, Mais und Baumwolle, als der bekannte 1,1-Dimethyl-3-(4-fluor-phenyl)-harnstoff.

Beispiel B

Pre-emergence-Test

Lösungsmittel: 5 Gewichtsteile Aceton
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen-Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt die gemäß dem Herstellungsbeispiel (1) erhaltene Verbindung stärkere Wirkung gegen Unkräuter und wesentlich bessere Verträglichkeit gegenüber Kulturpflanzen, wie z.B. Gerste und Baumwolle, als der bekannte 1,1-Dimethyl-3-(4-fluor-phenyl)-harnstoff.

## Ansprüche

1. Difluorphenylharnstoffe der Formel (I),

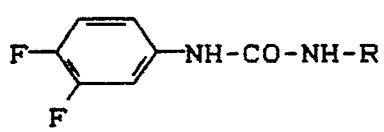

(I)

in welcher
R für jeweils geradkettiges oder verzweigtes Alkyl, Alkenyl oder Alkinyl mit jeweils bis zu 6 Kohlenstoffatomen, für jeweils gegebenenfalls durch Alkylreste substituiertes Cycloalkyl oder Cycloalkylalkyl mit bis zu 6 Kohlenstoffatomen im Cycloalkylteil und gegebenenfalls bis zu 4 Kohlenstoffatomen im Alkylteil, oder für jeweils geradkettiges oder verzweigtes Alkoxy oder Alkenyloxy mit jeweils bis zu 6 Kohlenstoffatomen steht.

2. Difluorphenylharnstoff der Formel (I) gemäß Anspruch 1, dadurch gekennzeichnet, daß darin
R für jeweils geradkettiges oder verzweigtes Alkyl, Alkenyl oder Alkinyl mit jeweils bis zu 4 Kohlenstoffatomen, für jeweils gegebenenfalls durch $C_1$- $C_4$-Alkylreste substituiertes Cycloalkyl oder Cycloalkylalkyl mit bis zu 6 Kohlenstoffatomen im Cycloalkylteil und gegebenenfalls 1 oder 2 Kohlenstoffatomen im Alkylteil, oder für jeweils geradkettiges oder verzweigtes Alkoxy oder Alkenyloxy mit jeweils bis zu 4 Kohlenstoffatomen steht.

3. Difluorphenylharnstoffe der Formel (I) gemäß Anspruch 1, dadurch gekennzeichnet, daß darin
R für Methyl, Ethyl, Propyl, Isopropyl, Allyl, Propargyl oder Cyclopropyl steht.

4. Verfahren zur Herstellung von Difluorphenylharnstoffen der Formel (I) gemäß Anspruch 1, dadurch gekennzeichnet, daß man Difluorphenylisocyanat der Formel (II)

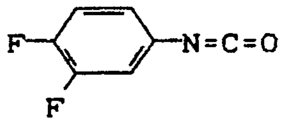

(II)

mit Aminen der Formel (III)

$$H_2N-R \qquad (III)$$

in welcher

R die oben angegebene Bedeutung hat,

gegebenenfalls in Gegenwart eines Verdünnungsmittels umsetzt.

5. Herbizide Mittel, gekennzeichnet durch einen Gehalt an mindestens einem Difluorphenylharnstoff der Formel (I) gemäß Anspruch 1.

6. Verfahren zur Bekämpfung von Unkräutern, dadurch gekennzeichnet, daß man Difluorphenylharnstoffe der Formel (I) gemäß Anspruch 1 auf die Unkräuter und/oder ihren Lebensraum einwirken läßt.

7. Verwendung von Difluorphenylharnstoffen der Formel (1) gemäß Anspruch 1 zur Bekämpfung von Unkräutern.

8. Verfahren zur Herstellung von herbiziden Mitteln, dadurch gekennzeichnet, daß man Difluorphenylharnstoffe der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 90123611.7

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int Cl 5) |
|---|---|---|---|
| D,Y | DE - A1 - 3 800 269 (BAYER) * Ansprüche * -- | 1-8 | C 07 C 275/30<br>A 01 N 47/30 |
| D,Y | DE - A - 1 905 599 (CIBA) * Ansprüche 1,2 * -- | 1,5-8 | |
| Y | DE - A - 2 223 839 (DIAMOND SHAMROCK) * Ansprüche * -- | 1-8 | |
| Y | GB - A - 1 410 493 (THE ANSUL COMPANY) * Ansprüche * -- | 1-8 | |
| Y | GB - A - 2 068 955 (SHELL INTERNATIONALE) * Ansprüche * -- | 1-3, 5-8 | |
| Y | GB - A - 1 112 760 (ARMOUR AND COMPANY) * Anspruch 1 * -- | 1 | RECHERCHIERTE SACHGEBIETE (Int Cl 5) |
| Y | EP - A2 - 0 103 919 (ANIC) * Beispiel 9 * ---- | 1 | C 07 C 275/00<br>A 01 N |

Der vorliegende Recherchenbericht wurde für alle Patentansprüche erstellt

| Recherchenort | Abschlußdatum der Recherche | Prüfer |
|---|---|---|
| WIEN | 04-03-1991 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82